(19) Europäisches Patentamt
European Patent Office
Office européen des brevets

(11) Publication number: **0 386 951 A2**

(12) # EUROPEAN PATENT APPLICATION

(21) Application number: **90302236.6**

(22) Date of filing: **02.03.90**

(51) Int. Cl.5: **A61K 37/02, A61K 9/08, A61K 47/04**

(30) Priority: **06.03.89 US 319248**

(43) Date of publication of application:
**12.09.90 Bulletin 90/37**

(84) Designated Contracting States:
**AT BE CH DE DK ES FR GB GR IT LI LU NL SE**

(71) Applicant: **ELI LILLY AND COMPANY**
**Lilly Corporate Center**
**Indianapolis Indiana 46285(US)**

(72) Inventor: **Inman, Eugene Lee**
**8061 Castle Cove Road**
**Indianapolis, Indiana 46256(US)**
Inventor: **Kirsch, Lee Edwin**
**7630 Camelback Drive**
**Indianapolis, Indiana 46250(US)**

(74) Representative: **Hudson, Christopher Mark et al**
**Erl Wood Manor**
**Windlesham Surrey GU20 6PH(GB)**

(54) **An improved diluent formulation for daptomycin.**

(57) Improved formulations for the lipopeptide antibiotic, daptomycin, are provided. Also provided are an improved kit for parenteral administration of daptomycin and a buffered pharmaceutical composition comprising daptomycin and a parenterally-acceptable buffer.

EP 0 386 951 A2

## AN IMPROVED DILUENT FORMULATION FOR DAPTOMYCIN

In the field of antibiotics, particularly in the field of lipopeptide antibiotics, searches are constantly ongoing for new and improved formulations for delivery of the active ingredients into the subject under antimicrobial treatment. In this regard, it is of paramount importance that the formulation of the active ingredient be one that allows the drug to be bioavailable, while at the same time inhibiting, or at least not contributing to, premature degradation of the active ingredient. Further, it is of equal, if not greater, importance that the formulation itself be one that does not unacceptably enhance toxicity or any other untoward effect to the subject.

The present invention provides a solution to the problem of the premature "in solution" degradation of the known lipopeptide antibiotic daptomycin. Daptomycin is represented by the following structural Formula (I)

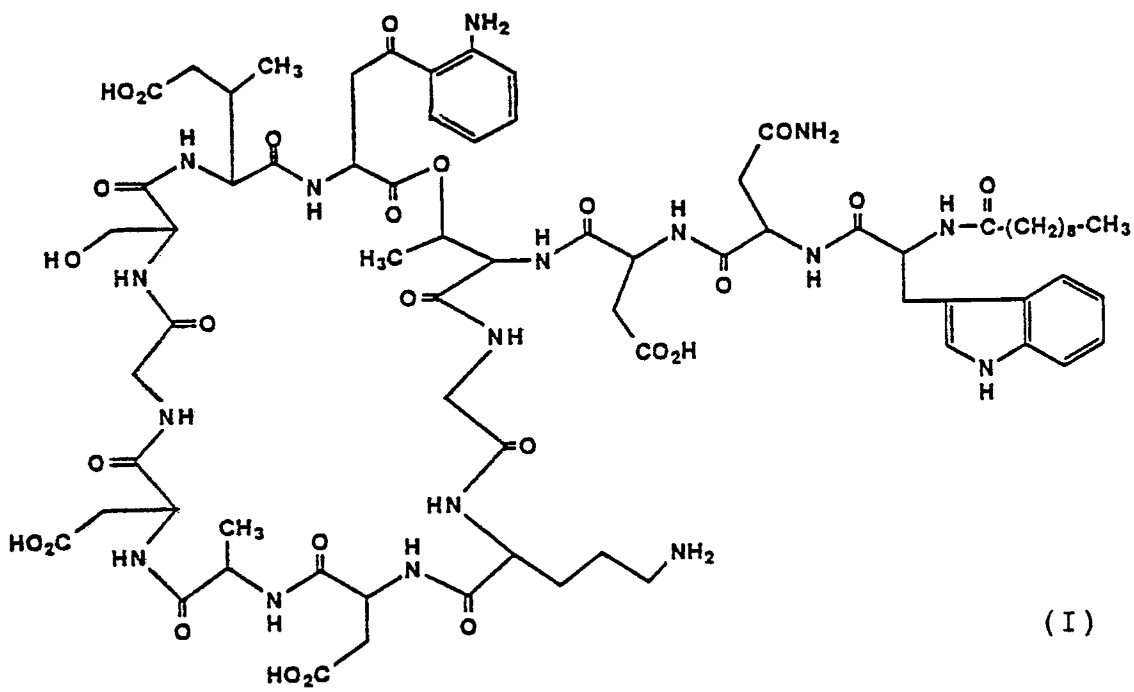

(I)

and is described in U.S. Patent No. 4,537,717. Stabilized daptomycin formulations are provided herein which allow the antibiotic to be prepared for parenteral administration in commonly used diluents such as 5% dextrose solution, with minimal degradation. Following the realization that daptomycin, when dissolved in a 5% dextrose solution, undergoes 15-20% degradation in 24 h at 25° C or in 7 days at 5° C, it was discovered that formulations with sufficient buffer capacity in the pH range 6-8 reduce the degradation to approximately 1%.

The present invention provides stabilized formulations of daptomycin (I) which possess sufficient buffer capacity to allow for storage of daptomycin solutions in 5% dextrose without significant degradation.

It has recently been discovered that daptomycin, when dissolved in 5% dextrose solution, undergoes 15-20% degradation into two unidentified products in 24 h at 25° C or 7 days at 5° C. The degradation, which also was found to be unique to reducing sugars and especially aldoses (e.g., glucose, mannose, galactose, arabinose, etc.), appears to be pH related and is accompanied by a significant loss of antibiotic potency. As an illustration of this phenomenon, the following study was conducted:

First, 8 vials of daptomycin (100 mg each) were reconstituted to a concentration of 1 mg/ml with normal saline and 5% dextrose (4 each). Next, the solutions were stored at room temperature (approximately 25° C) and refrigerated temperatures and assayed at the 6 h and 24 h timepoints.

| Time (hrs.) | Temp. | pH | Clarity (NTU) | Visual | Potency (mg/mL) | Total Appearance of Degradation Products |
|---|---|---|---|---|---|---|
| 0.9% NaCl: | | | | | | |
| Initial | -- | 4.2 | 1.0 | ACPL* | 1.026 | 5.2 |
| 6 | A | 4.2 | 0.9 | 1111** | 1.025 | 5.1 |
| | B | 4.2 | 0.8 | 1111 | 1.025 | 5.6 |
| 24 | A | 4.2 | 0.9 | 1111 | 1.019 | 5.6 |
| | B | 4.2 | 0.7 | 1111 | 1.002 | 7.5 |
| 5% Dextrose: | | | | | | |
| Initial | -- | 4.5 | 1.7 | ACPL | 1.023 | 8.9 |
| 6 | A | 4.5 | 1.6 | 1111 | 1.001 | 12.6 |
| | B | 4.5 | 0.7 | 1111 | 0.963 | 16.6 |
| 24 | A | 4.5 | 1.5 | 1111 | 0.975 | 16.2 |
| | B | 4.5 | 1.1 | 1111 | 0.848 | 24.9 |

*Inspection for appearance, color, package and clarity.
**1 indicates no change from initial.

In sum, solutions of daptomycin and dextrose held at refrigerated temperature demonstrated a potency loss of about 5% at the 24 h timepoint, while solutions held at room temperature showed a 17% loss over the same time period.

Further, a series of degradation kinetic studies demonstrate that the rate of appearance of degradation products is pH-dependent. For example, the appearance half-life for the degradation products at 25° C and pH 4.5 was 6 h, while at 25° C and pH 7.0, the half-life was 63 h.

The nature of the degradation products has as yet not been determined; although, as indicated by the loss in potency of unstabilized solutions of daptomycin, these products exhibit far less antibiotic activity than the present antibiotic.

The stabilized formulations provided herein are suitable for administration by the parenteral routes and comprise an antibiotically effective amount of daptomycin, a pharmaceutically acceptable buffer and a pharmaceutically acceptable diluent. In addition, the formulations can contain excipients.

Pharmaceutically acceptable buffers which can be used include sodium or potassium salts of phosphoric acid, boric acid, citric acid, carbonic acid, hydroxide, and the like; tris(hydroxymethyl)aminomethane (TRIS® buffer), amino acids, and like buffers.

Diluents which can be used include water for injection, 5% dextrose, 0.9% saline, Ringer's solution and like commonly used diluents for the parenterally administered antibiotics.

Excipients which can be used include, for example, mannitol, tonicity modifiers (e.g., sodium chloride or glycerol), preservatives and solubilizing agents. Further examples of acceptable diluents and excipients can be found in Remington's Pharmaceutical Sciences, 17th Ed., A. R. Gennaro, Ed., Mack Publishing Co., Easton, PA, 1985, incorporated herein by reference; particularly relevant are pages 1518-1552.

Preferred buffers of this invention are dibasic sodium phosphate and TRIS® (tris(hydroxymethyl)-aminomethane buffer.

Thus, in broadest terms, the present invention provides an improved formulation comprising daptomycin and a parenterally-acceptable buffer possessing sufficient buffer capacity to maintain the pH of the reconstituted daptomycin between a pH of between about 6.0 to about 8.0.

Specific examples of preferred parenterally acceptable buffer systems are provided in the following Table 1:

Table 1

| | |
|---|---|
| Formulation A | Isotonic Sodium Phosphate Dibasic Solution (8.73 mg/mL) |
| Formulation B | Isotonic Sodium Phosphate Dibasic Solution (15.76 mg/mL) |
| Formulation C | TRIS® (Tris(hydroxymethyl)aminomethane) Solution (3.30 mg/mL) |
| Formulation D | TRIS® (Tris(hydroxymethyl)aminomethane) Solution (3.74 mg/mL) |
| Formulation E | TRIS® (Tris(hydroxymethyl)aminomethane) Solution (4.96 mg/mL) |

Formulations comprising the buffers in Table 1 were tested by reconstituting freeze-dried daptomycin (150 mg) and 50 mg mannitol (50 mg) in 10 mL of each buffer, then diluting the resultant solution in 100 mL of 5% dextrose solutions. The effectiveness of the buffered diluents in suppressing the appearance of degradation products is shown below in Table 2:

Table 2:

| Summary Stability Results . Percent Increase in Daptomycin Degradation Products at 1 day storage (25° C) and 7 days storage (5° C). | | | |
|---|---|---|---|
| | | % Increase in Dextrose Related Degradation Products | |
| Formulation | pH | 1 day (25° C) | 7 days (5° C) |
| 5% dextrose (control) | 4.4 | 16.9 | 17.3 |
| A | 6.9 | 1.1 | 1.1 |
| B | 7.3 | 0.6 | 0.5 |
| C | 6.9 | 1.8 | 0.8 |
| D | 7.4 | 0.5 | 0.4 |
| E | 7.9 | 0.3 | 0.3 |

Further examples of buffer systems meant by the term "pharmaceutically-acceptable buffer" include sodium or potassium salts of phosphoric acid, boric acid, citric acid, carbonic acid, hydroxide, and the like; tris(hydroxymethyl)aminomethane (TRIS® buffer) and all amino acids and like buffers.

In the case where sodium phosphate dibasic is used as buffer, it is preferred that the concentration be in the range of about 0.02 mMolar to about 0.13 mMolar, most preferably about 0.05 mMolar to about 0.10 mMolar. One skilled in the art will appreciate that the amount of buffer necessary to maintain the pH in the desired range is directly related to the amount of daptomycin present in solution. Thus, the above ranges are preferred when daptomycin is present in a concentration of about 6 x $10^{-4}$ mMolar.

The following is a nonexhaustive list of examples of unit dose formulations for daptomycin:

| | Ingredient | Amount |
|---|---|---|
| 1) | Daptomycin | 150 mg |
| | Mannitol | 50 mg |
| | Isotonic Sodium Phosphate Dibasic Solution (8.73 mg/ml) | 10 ml |
| | Dextrose (5%) | 90 ml |
| 2) | Daptomycin | 200 mg |
| | Mannitol | 66 mg |
| | Isotonic Sodium Phosphate Dibasic Solution (8.73 mg/ml) | 13 ml |
| | Dextrose (5%) | 87 ml |
| 3) | Daptomycin | 250 mg |
| | Mannitol | 82 mg |
| | Isotonic Sodium Phosphate Dibasic Solution (8.73 mg/ml) | 16 ml |
| | Dextrose (5%) | 84 ml |
| 4) | Daptomycin | 300 mg |
| | Mannitol | 100 mg |
| | Isotonic Sodium Phosphate Dibasic Solution (8.73 mg/ml) | 20 ml |
| | Dextrose (5%) | 80 ml |

| | | |
|---|---|---|
| 5) | Daptomycin | 150 mg |
| | Mannitol | 50 mg |
| | Isotonic Sodium Phosphate Dibasic Solution (15.76 mg/ml) | 10 ml |
| | Dextrose (5%) | 90 ml |
| 6) | Daptomycin | 200 mg |
| | Mannitol | 60 mg |
| | Isotonic Sodium Phosphate Dibasic Solution (15.76 mg/ml) | 13 ml |
| | Dextrose (5%) | 87 ml |
| 7) | Daptomycin | 250 mg |
| | Mannitol | 82 mg |
| | Isotonic Sodium Phosphate Dibasic Solution (15.76 mg/ml) | 16 ml |
| | Dextrose (5%) | 84 ml |
| 8) | Daptomycin | 300 mg |
| | Mannitol | 100 mg |
| | Isotonic Sodium Phosphate Dibasic Solution (15.76 mg/ml) | 20 ml |
| | Dextrose (5%) | 80 ml |
| 9) | Daptomycin | 150 mg |
| | Mannitol | 50 mg |
| | Tris(hydroxymethyl)amminomethane Solution (3.30 mg/ml) | 10 ml |
| | Dextrose (5%) | 90 ml |

| | | |
|---|---|---|
| 10) | Daptomycin | 200 mg |
| | Mannitol | 66 mg |
| | Tris(hydroxymethyl)amminomethane Solution (3.30 mg/ml) | 13 ml |
| | Dextrose (5%) | 87 ml |
| 11) | Daptomycin | 250 mg |
| | Mannitol | 82 mg |
| | Tris(hydroxymethyl)amminomethane Solution (3.30 mg/ml) | 16 ml |
| | Dextrose (5%) | 84 ml |
| 12) | Daptomycin | 300 mg |
| | Mannitol | 100 mg |
| | Tris(hydroxymethyl)amminomethane Solution (3.30 mg/ml) | 20 ml |
| | Dextrose (5%) | 80 ml |
| 13) | Daptomycin | 150 mg |
| | Mannitol | 50 mg |
| | Tris(hydroxymethyl)amminomethane Solution (3.74 mg/ml) | 10 ml |
| | Dextrose (5%) | 90 ml |
| 14) | Daptomycin | 200 mg |
| | Mannitol | 66 mg |
| | Tris(hydroxymethyl)amminomethane Solution (3.74 mg/ml) | 13 ml |
| | Dextrose (5%) | 87 ml |

| | | |
|---|---|---|
| 15) | Daptomycin | 250 mg |
| | Mannitol | 82 mg |
| | Tris(hydroxymethyl)amminomethane Solution (3.74 mg/ml) | 16 ml |
| | Dextrose (5%) | 84 ml |
| 16) | Daptomycin | 300 mg |
| | Mannitol | 100 mg |
| | Tris(hydroxymethyl)amminomethane Solution (3.74 mg/ml) | 20 ml |
| | Dextrose (5%) | 80 ml |
| 17) | Daptomycin | 150 mg |
| | Mannitol | 50 mg |
| | Tris(hydroxymethyl)amminomethane Solution (4.96 mg/ml) | 10 ml |
| | Dextrose (5%) | 90 ml |
| 18) | Daptomycin | 200 mg |
| | Mannitol | 66 mg |
| | Tris(hydroxymethyl)amminomethane Solution (4.96 mg/ml) | 13 ml |
| | Dextrose (5%) | 87 ml |
| 19) | Daptomycin | 250 mg |
| | Mannitol | 82 mg |
| | Tris(hydroxymethyl)amminomethane Solution (4.96 mg/ml) | 16 ml |
| | Dextrose (5%) | 84 ml |
| 20) | Daptomycin | 300 mg |
| | Mannitol | 100 mg |
| | Tris(hydroxymethyl)amminomethane Solution (4.96 mg/ml) | 20 ml |
| | Dextrose (5%) | 80 ml |

This aspect of the present invention may be practiced in two ways. First, the daptomycin may be purchased in a lyophilized form and reconstituted prior to use with a buffered diluent as discussed above.

Alternatively, daptomycin may be packaged with a suitable amount of buffer so that upon reconstitution with a (non-buffered) diluent, the resulting reconstituted formulation will also have a pH in the range of about 6 to about 8.

As another aspect of the present invention, there is provided a pharmaceutical composition comprising an antibiotically-effective amount of the antibiotic daptomycin, combined with a parenterally-acceptable buffer in sufficient quantity so as to afford a solution pH of about 6.0 to about 8.0 when diluted with one or more pharmaceutically-acceptable diluents and excipients.

As a further aspect of the present invention, there is provided a parenteral formulation of daptomycin, which comprises admixing daptomycin with one or more pharmaceutically-acceptable diluents and excipients characterized in that a sufficient quantity of a parenterally-acceptable buffer is added to maintain the formulation at a pH of between about 6.0 and about 8.0.

## Claims

1. A parenteral formulation which comprises as an active ingredient daptomycin associated with a sufficient amount of a parenterally-acceptable buffer to maintain the pH between about 6.0 and about 8.0, and one or more pharmaceutically-acceptable diluents or excipients.

2. A formulation as claimed in claim 1, wherein the buffer is sodium phosphate dibasic.

3. A formulation as claimed in claim 1, wherein the buffer is tris(hydroxymethyl)aminomethane.

4. A formulation as claimed in claim 2, wherein the buffer sodium phosphate dibasic is present in the concentration of about 0.02 to about 0.13 mMolar.

5. A formulation as claimed in claim 3, wherein the buffer tris(hydroxymethyl)aminomethane is present in a concentration of about 0.015 to 0.03 mMolar.

6. A formulation as claimed in claim 4, which comprises

| | | |
|---|---|---|
| (a) | Daptomycin | 150 mg |
| | Mannitol | 50 mg |
| | Isotonic Sodium Phosphate Dibasic Solution (8.73 mg/ml) | 10 ml |
| | Dextrose (5%) | 90 ml; |

| | | |
|---|---|---|
| (b) | Daptomycin | 200 mg |
| | Mannitol | 66 mg |
| | Isotonic Sodium Phosphate Dibasic Solution (8.73 mg/ml) | 13 ml |
| | Dextrose (5%) | 87 ml; |
| (c) | Daptomycin | 250 mg |
| | Mannitol | 82 mg |
| | Isotonic Sodium Phosphate Dibasic Solution (8.73 mg/ml) | 16 ml |
| | Dextrose (5%) | 84 ml; |
| (d) | Daptomycin | 300 mg |
| | Mannitol | 100 mg |
| | Isotonic Sodium Phosphate Dibasic Solution (8.73 mg/ml) | 20 ml |
| | Dextrose (5%) | 80 ml; |
| (e) | Daptomycin | 150 mg |
| | Mannitol | 50 mg |
| | Isotonic Sodium Phosphate Dibasic Solution (15.76 mg/ml) | 10 ml |
| | Dextrose (5%) | 90 ml; |

| | | |
|---|---|---|
| (f) | Daptomycin | 200 mg |
| | Mannitol | 60 mg |
| | Isotonic Sodium Phosphate Dibasic Solution (15.76 mg/ml) | 13 ml |
| | Dextrose (5%) | 87 ml; |
| (g) | Daptomycin | 250 mg |
| | Mannitol | 82 mg |
| | Isotonic Sodium Phosphate Dibasic Solution (15.76 mg/ml) | 16 ml |
| | Dextrose (5%) | 84 ml; |
| (h) | Daptomycin | 300 mg |
| | Mannitol | 100 mg |
| | Isotonic Sodium Phosphate Dibasic Solution (15.76 mg/ml) | 20 ml |
| | Dextrose (5%) | 80 ml; |
| (i) | Daptomycin | 150 mg |
| | Mannitol | 50 mg |
| | Tris(hydroxymethyl)aminomethane Solution (3.30 mg/ml) | 10 ml |
| | Dextrose (5%) | 90 ml; |

| | | | |
|---|---|---|---|
| (j) | Daptomycin | 200 | mg |
| | Mannitol | 66 | mg |
| | Tris(hydroxymethyl)aminomethane Solution (3.30 mg/ml) | 13 | ml |
| | Dextrose (5%) | 87 | ml; |
| (k) | Daptomycin | 250 | mg |
| | Mannitol | 82 | mg |
| | Tris(hydroxymethyl)aminomethane Solution (3.30 mg/ml) | 16 | ml |
| | Dextrose (5%) | 84 | ml; |
| (l) | Daptomycin | 300 | mg |
| | Mannitol | 100 | mg |
| | Tris(hydroxymethyl)aminomethane Solution (3.30 mg/ml) | 20 | ml |
| | Dextrose (5%) | 80 | ml; |
| (m) | Daptomycin | 150 | mg |
| | Mannitol | 50 | mg |
| | Tris(hydroxymethyl)aminomethane Solution (3.74 mg/ml) | 10 | ml |
| | Dextrose (5%) | 90 | ml; |

| | | |
|---|---|---|
| (n) | Daptomycin | 200 mg |
| | Mannitol | 66 mg |
| | Tris(hydroxymethyl)aminomethane Solution (3.74 mg/ml) | 13 ml |
| | Dextrose (5%) | 87 ml; |
| (o) | Daptomycin | 250 mg |
| | Mannitol | 82 mg |
| | Tris(hydroxymethyl)aminomethane Solution (3.74 mg/ml) | 16 ml |
| | Dextrose (5%) | 84 ml; |
| (p) | Daptomycin | 300 mg |
| | Mannitol | 100 mg |
| | Tris(hydroxymethyl)aminomethane Solution (3.74 mg/ml) | 20 ml |
| | Dextrose (5%) | 80 ml; |
| (q) | Daptomycin | 150 mg |
| | Mannitol | 50 mg |
| | Tris(hydroxymethyl)aminomethane Solution (4.96 mg/ml) | 10 ml |
| | Dextrose (5%) | 90 ml; |

| | | |
|---|---|---|
| (r) | Daptomycin | 200 mg |
| | Mannitol | 66 mg |
| | Tris(hydroxymethyl)aminomethane Solution (4.96 mg/ml) | 13 ml |
| | Dextrose (5%) | 87 ml; |
| (s) | Daptomycin | 250 mg |
| | Mannitol | 82 mg |
| | Tris(hydroxymethyl)aminomethane Solution (4.96 mg/ml) | 16 ml |
| | Dextrose (5%) | 84 ml; or |
| (t) | Daptomycin | 300 mg |
| | Mannitol | 100 mg |
| | Tris(hydroxymethyl)aminomethane Solution (4.96 mg/ml) | 20 ml |
| | Dextrose (5%) | 80 ml. |

7. A therapeutic kit for the preparation of a parenteral formulation of the antibiotic daptomycin, said kit comprising at least two containers, one of which contains daptomycin; and a second of which contains a buffer capable of maintaining the pH of a solution of daptomycin in the first container between a pH of about 6.0 and about 8.0 when mixed, each of said first and second containers comprising one or more pharmaceutically acceptable carriers or excipients as appropriate.

8. A pharmaceutical composition comprising an antibiotically-effective amount of the antibiotic daptomycin, combined with a parenterally-acceptable buffer in sufficient quantity so as to afford a solution pH of about 6.0 to about 8.0 when diluted with one or more pharmaceutically-acceptable diluents and excipients.

9. A process for preparing a parenteral formulation of daptomycin, which comprises admixing daptomycin with one or more pharmaceutically-acceptable diluents or excipients, characterized in that a sufficient quantity of a parenterally-acceptable buffer is added to maintain the formulation at a pH of between about 6.0 and about 8.0.